# EUROPÄISCHE PATENTSCHRIFT

(11) **EP 3 115 005 B1**
(45) Veröffentlichungstag und Bekanntmachung des Hinweises auf die Patenterteilung: **17.10.2018**
(21) Anmeldenummer: 16178917.7
(22) Anmeldetag: 11.07.2016
(51) Int. Cl.: A61B 17/22, A61H 23/00, A61B 17/225

(54) **VORRICHTUNG ZUR ERZEUGUNG VON STOSSWELLEN**
DEVICE FOR GENERATING IMPACT WAVES
DISPOSITIF DE PRODUCTION D'ONDES DE CHOC

(30) Priorität: 10.07.2015 DE 102015008949
(43) Veröffentlichungstag der Anmeldung: 11.01.2017
(73) Patentinhaber: CellVitalis Holding GmbH, 8280 Kreuzlingen (CH)
(72) Erfinder: Möbius, Andreas, 78464 Konstanz (DE); Brinkmann, Ralph, 8280 Kreuzlingen (CH)
(74) Vertreter: Molnia, David

(56) Entgegenhaltungen:
- DE-C2- 19 718 511
- US-A1- 2002 193 709
- US-A1- 2013 345 600
- US-A1- 2014 257 144

## Beschreibung

### Technisches Gebiet und Anwendung

Die Erfindung beschreibt eine Vorrichtung zur Erzeugung von Stoßwellen in einem flüssigen Medium, insbesondere zur Erzeugung von elektro-hydraulischen Stoßwellen, mit einem auswechselbaren Applikatorkopf. Die elektro-hydraulische Stoßwellenerzeugung ist dadurch charakterisiert, dass über zwei Zündelektrodenspitzen in einem flüssigen Medium eine Hochspannung entladen wird. Bei der Entladung kommt es zwischen den zwei Spitzen zur Ausbildung eines Stromkanals, der zunächst zur starken Erhitzung des flüssigen Mediums und dadurch danach zur Ausbildung einer Plasmablase führt. Die Ausdehnung und der anschließende Kollaps der Plasmablase führen im flüssigen Medium zu einem hohen Druckanstieg gefolgt von einem Druckabfall. Es entsteht eine Druckwelle, die sogenannte Stoßwelle, mit kurzem positiven Druckanteil, gefolgt von einem zeitlich längeren negativen Zuganteil, die sich in der Flüssigkeit ausbreitet. Durch einen die Flüssigkeit umgebenden metallischen Reflektor kann die Stoßwelle über eine durchlässige Membran nach außen reflektiert werden. Der erfindungsgemäße Applikatorkopf umfasst folglich einen Reflektor, das flüssige Medium, zwei Zündelektrodenspitzen und eine Membran sowie eine elektrisch-mechanische Koppelmöglichkeit. Der erfindungsgemäße Applikatorkopf ist derart ausgestaltet, dass er mit einem Handapparat elektrisch und mechanisch lösbar verbunden ist. Erfindungsgemäß soll der Applikatorkopf ein einfaches "glühbirnenartiges" Auswechseln ermöglichen und somit dem Anwender ermöglichen mehrere gleichartige oder unterschiedliche Applikatorköpfe verwenden zu können und platzsparend zu verstauen.

Stoßwellen werden in der Human- und Veterinärmedizin für unterschiedliche Zwecke eingesetzt. Medizinische Anwendungen dieser Vorrichtungen sind die Behandlung von Hart- und Weichteilgewebe, insbesondere zur Anregung von Knochenwachstum und Stimulation der Heilung bei orthopädischen, schmerzhaften Erkrankungen (z.B. Tennisellbogen, Fersensporn, Kalkschulter), therapeutische Behandlung von Nerven, Muskeln und anderen Weichteilstrukturen, zur Durchblutungsanregung sowie die Behandlung von akuten und chronischen Entzündungen im menschlichen oder tierischen Gewebe.

### Stand der Technik

Aus dem Stand der Technik sind Stoßwellenapplikatoren bekannt, die über eine elektrische Hochspannungsverbindung mit einer Steuereinheit verbunden sind. Der Stand der Technik kennt ferner Steuereinheiten, die als Tischgeräte oder mobile, transportable Geräte ausgebildet sind.

Herkömmliche Stoßwellen-Applikatoren, insbesondere elektro-hydraulische, umfassen einen Handapparat, der mit der Steuereinheit fest oder lösbar verbunden ist. Der Handapparat enthält unlösbar einen Applikatorkopf bestehend aus einem Reflektor mit flüssigem Medium, der durch eine Membran verschlossen ist sowie zwei Elektrodenspitzen. Das Auswechseln des Stoßwellenapplikators kann nur in der Weise vorgenommen werden, dass auch die elektrische und mechanische Verbindung, zumeist als Kabel ausgebildet, als eine Einheit mitsamt dem Applikatorkopf und Handapparat von der Steuereinheit gelöst wird. Die Verschleißteile dieser Systeme bestehen mindestens aus den zwei Elektrodenspitzen und zumeist auch in dem die Spitzen umgebenden flüssigen Medium. Da die Elektrodenspitzen am spannungszuführenden Kabel so angekoppelt sein müssen, dass der durch die Spannung entladene Strom möglichst ungehindert fließen kann, besteht die Verbindung bei herkömmlichen elektrohydraulischen Systemen aus Schraub-, Klemm- und/oder Lötverbindungen, wodurch der Applikatorkopf unlösbar mit dem Handapparat, dem Kabel und der Kupplung zur Versorgungseinheit verbunden ist.

Um Verschleißteile wie die Elektrodenspitzen und das die Spitzen umgebende flüssige Medium auszuwechseln, muss folglich der gesamte Handapparat mit Anschlusskabel ausgewechselt werden. Dadurch, dass auch die Teile, die nicht zwingend verschleißen (Handapparat, Kabel, Kupplung), für das Auswechseln der Verschleißteile immer mitversendet werden müssen, sind das Gewicht, der Platzbedarf und die Kosten für die Aufbewahrung, den Transport und den Versand der Teile, die häufig in großen Stückzahlen versendet werden müssen, erheblich. Zudem ist das Auswechseln der Verschleißteile nur mithilfe von Spezialwerkzeug möglich.

US 2014/0257144 A1 zeigt einen Generator für schnell gepulste elektrohydraulische (EH) Stosswellen und ein Verfahren zur Durchführung von medizinischen und kosmetischen Behandlungen.

DE 197 18 511 C2 zeigt ein Gerät zur Applikation von akustischen Stoßwellen.

US 2013/0345600 A1 zeigt ein Verfahren zur Erhöhung der Lebensdauer von Elektroden in Geräten für die extrakorporale Stoßwellentherapie (ESWT).

US 2002/0193709 A1 zeigt eine Vorrichtung zum Verabreichen von akustischen Stoßwellen mit einer entfernbaren und austauschbaren Komponente eines Datenspeichermediums.

### Darstellung der Erfindung

Ausgehend von dem bekannten Stand der Technik ist es eine Aufgabe der vorliegenden Erfindung, eine verbesserte Vorrichtung zur Erzeugung von Stoßwellen bereitzustellen, wobei das Auswechseln des Applikatorkopfes und der in diesem enthaltenen Verschleißteile vereinfacht wird.

Die Aufgabe wird durch eine Vorrichtung zur Erzeugung von Stoßwellen mit den Merkmalen des Anspruchs 1 gelöst. Vorteilhafte Weiterbildungen ergeben sich aus den Unteransprüchen.

Entsprechend wird eine Vorrichtung zur Erzeugung von Stoßwellen für die Behandlung am menschlichen oder tierischen Körper vorgeschlagen, welche einen auswechselbaren Applikatorkopf zur Erzeugung von Stoßwellen für die Behandlung am menschlichen oder tierischen Körper besitzt, wobei der Applikatorkopf eine Reflexions- oder Fokussierungseinrichtung, zwei Elektrodenspitzen zur Erzeugung einer Funkenstrecke sowie eine Membran, die mit der Reflexions- oder Fokussierungseinrichtung ein flüssiges Medium einschließt, das geeignet ist, beim Anlegen einer Spannung zwischen 1 kV und 30 kV ein Plasma zu erzeugen, umfasst. Der Applikatorkopf ist lösbar mit einem Handapparat elektrisch und mechanisch verbunden. Erfindungsgemäß kann das Auswechseln des Applikatorkopfes mittels einer im Handapparat untergebrachten elektrisch-mechanischen, steuerbaren Verriegelungsvorrichtung verhindert und freigegeben werden.

Hierdurch wird auch ein vom Applikatorkopf lösbarer Handapparat bereitgestellt, der mit Verbindungskabel und Steckverbindung an der Steuereinheit verbleiben kann oder von dieser separat lösbar gestaltet ist. Es können unterschiedliche oder gleichartige Applikatorköpfe mit dem Handapparat lösbar verbunden werden, so dass diese bspw. ebenfalls separat verstaut werden können. Die Applikatorköpfe können hierbei unterschiedliche Reflektorgeometrien, Leistungsdaten und Dimensionen aufweisen, die den jeweilig gewünschten Anwendungen entsprechen. Hierbei können bspw. unterschiedlich große Zielgebiete und Eindringtiefen wünschenswert sein. So ist bspw. ein einfaches Verfahren verschiedene Abstrahlungscharakteristiken zu erzeugen, das Verschieben des Elektrodenspalts aus dem primären Fokus eines halbellipsoidalen Reflektors, welches zu einer unscharfen und verzerrten Abbildung des primären Fokus im sekundären Fokus führt.

Speziell für die mobile Anwendung ist es vorteilhaft für unterschiedliche Therapieanforderungen geeignete Applikatoren verwenden zu können. Dabei ist es vorteilhaft, wenn die auswechselbare Einheit nur noch aus dem Applikatorkopf besteht und nicht mehr wie herkömmlich aus Handapparat, Verbindungskabel und elektrisch-mechanischer Steckverbindung. Hierfür ist jetzt die wechselbare Einheit platzsparend und leichtgewichtig ausgebildet.

Der Applikatorkopf ist derart gestaltet, dass er eine lösbare elektrische und mechanische Verbindung zum Handapparat vorsieht. Er besteht aus dem Reflektor gefüllt mit einem flüssigen Medium, einer Zündvorrichtung mit zwei Elektrodenspitzen, die in den Reflektor gesteckt oder geschraubt werden kann, so dass die Elektrodenspitzen vom flüssigen Medium umgeben sind und einer Membran, die den Reflektor verschließt. Ferner kann der Applikatorkopf eine Zuleitung für die Beigabe oder Entnahme des flüssigen Mediums vorsehen. Mindestens einer der beiden verschleißenden Elektrodenspitzen ist inhärent, dass diese an einem Ende außerhalb des flüssigen Mediums elektrisch gut leitend angebunden und zum flüssigen Medium hin am anderen Ende mechanisch fest dichtend sein muss, um ein Auslaufen des wässrigen Mediums zu verhindern.

Dadurch, dass die lösbare Trennstelle nun in der Nähe der Elektrodenspitzen und des flüssigen Mediums - also der Verschleißteile - liegt, wird eine Reduktion der Größe und des Gewichts der wechselbaren Einheit ermöglicht. Ein Auswechseln des Applikatorkopfes wird dadurch erheblich erleichtert, dass nicht die gesamte Einheit bestehend aus Handapparat, Kabel und Kabelstecker beim Anwender ausgetauscht werden muss, sondern lediglich der Applikatorkopf einfach und schnell vom Anwender selbst ausgetauscht werden kann. Das ermöglicht zudem ein einfaches und schnelles Wechseln des Applikatorkopfes für unterschiedliche Behandlungszwecke durch den Anwender. Das vom Behandlungszweck unabhängige Kabel mit Stecker bleibt vor Ort an der Steuereinheit stets dasselbe. Da nur eine Steuereinheit/Versorgungseinheit und ein Handapparat für die unterschiedlichen Applikatorköpfe mit unterschiedlichen Stoßwellencharakteristiken benötigt werden, werden die Herstellungskosten, die Kosten für Transport und Versand, der Platz für die Aufbewahrung und das Gewicht der austauschbaren Applikatorköpfe minimiert, welche häufig in größeren Stückzahlen weltweit versendet werden müssen. Zudem kann das Lösen des Applikatorkopfes ohne die Verwendung von Werkzeug erfolgen. Trotz der lösbaren Verbindung zum einfachen und schnellen Auswechseln des Applikatorkopfes, werden eine sichere elektrisch leitfähige Verbindung und ein zuverlässig dichter Applikatorkopf gewährleistet. Die lösbare elektrische Verbindung ist so ausgebildet, dass sie eine hohe Steckzyklenzahl ermöglicht und es besteht weder beim Auswechseln, noch beim Transport der Applikatorköpfe die Gefahr von austretendem flüssigen Medium.

Für den Austausch der Verschleißteile im Applikatorkopf wird der über eine Schraubverbindung mit dem Applikatorkopf verbundene Zündkörper ohne Verwendung von Spezialwerkzeug ausgetauscht und der Applikatorkopf anschließend mit neuem Medium befüllt. Es entfällt das Lösen zahlreicher Schraubverbindungen mechanischer und elektrischer Art. Die Zündkörper können gesammelt und in einem getrennten Arbeitsschritt zur Wiederverwendung aufbereitet werden.

Weiterhin verfügt der Applikatorkopf über ein Kommunikations-Speicher-Medium, das es der Steuereinheit ermöglicht den Typ und die Leistungsdaten des Applikatorkopfes zu identifizieren. Das Kommunikations-Speicher-Medium kann derart gestaltet sein, dass es über eine elektrische oder kontaktlose Verbindung mit der Steuereinheit verbunden werden kann. Hierbei ist es vorteilhaft, dass die Verbindung über eine Radiofrequenz stattfindet. Eine eindeutige Identifizierung der Applikatorköpfe ist vorteilhaft, um eine Verwechslung der Typen oder Leistungsdaten auszuschließen oder die Abnutzung der Applikatorköpfe zu erkennen oder der Steuereinheit zu ermöglichen, unterschiedliche Leistungsdaten den unterschiedlichen Applikatorköpfen zuweisen zu können.

Bei der elektro-hydraulischen Stoßwellenerzeugung wird zwischen zwei Elektrodenspitzen in einem flüssigen Medium, das zumeist Wasser enthält, eine Hochspannung entladen und dabei ein sich explosionsartig ausbreitendes Plasma erzeugt, das wiederum eine Stoßwelle abgibt. Die hierbei verwendeten Spannungen reichen von wenigen Kilovolt bis zu 30 Kilovolt. Der Handapparat führt die Hochspannung dem Applikatorkopf zu. Um Gefahren durch berührbare Hochspannung für Anwender und Personen zu vermeiden, kennt der Stand der Technik die Vorschriften für elektrische Sicherheit, die vorschreiben, Maßnahmen zur Verhinderung der Berührbarkeit spannungsführender Teile zu implementieren. Ein Ansatz dazu ist, die lösbare Verbindung zu überwachen und zu verriegeln. Erfindungsgemäß muss diese Verriegelung im Handapparat erfolgen, so dass eine Ablösung des Applikatorkopfes nicht möglich ist, solange Hochspannung an berührbaren Teilen des Handapparates anliegt.

### Detaillierte Beschreibung bevorzugter Ausführungsbeispiele

Weitere Merkmale und Vorteile der Erfindung ergeben sich aus dem nachfolgend beschriebenen Ausführungsbeispiel in Verbindung mit den Figuren.

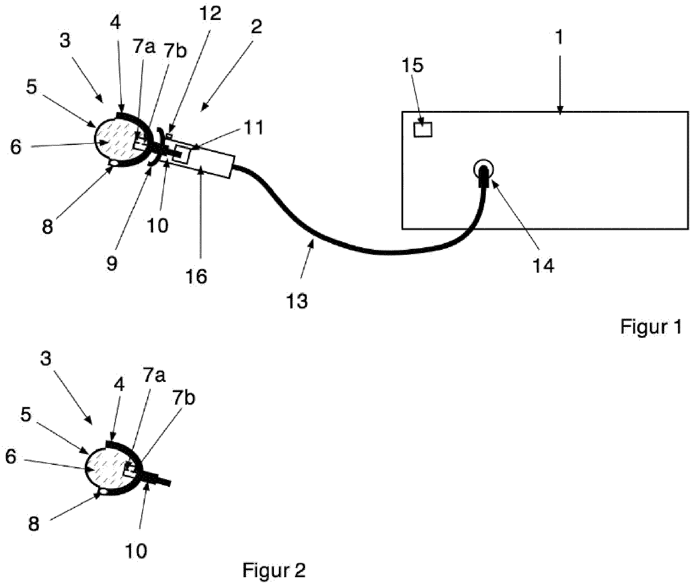

Dabei zeigt Figur 1 eine erfindungsgemäße Steuereinheit 1 in Form eines Tischgerätes mit einem Handapparat 2.

Der Handapparat 2 umfasst eine elektrisch-mechanische Verbindung 10, einen Handgriff 16, eine Applikatorkopfaufnahme 9 und eine Verriegelungsvorrichtung 11, welche den Applikatorkopf 3 aufnehmen kann und über das Verbindungskabel 13 und den Hochspannungsstecker 14 mit der Steuereinheit 1 lösbar verbunden ist. Der Handapparat sieht zudem einen Taster 12 für die Auslösung von Stoßwellen vor, der ebenfalls elektrisch lösbar mit der Steuereinheit 1 verbunden ist.

Figur 2 zeigt den Applikatorkopf 3, der den Reflektor 4, die Membran 5, die Elektroden 7a und 7b, ein Kommunikations-Speicher-Medium 8, eine elektrische Verbindung 10 sowie das flüssige Medium 6 umfasst, dass von der Membran 5 und dem Reflektor 4 eingeschlossen ist.

Die Steuereinheit sieht zudem eine drahtlose Schreib-Lesevorrichtung 15 vor, die das Kommunikations-Speicher-Medium 8 identifizieren, lesen und beschreiben kann.

### Bezuqszeichenliste:

- 1: Steuereinheit
- 2: Handapparat
- 3: Applikatorkopf
- 4: Reflektor
- 5: Membran
- 6: flüssiges Medium
- 7a: Elektrode
- 7b: Elektrode
- 8: Kommunikations-Speicher-Medium
- 9: mechanische Steckverbindung
- 10: elektrische Steckverbindung
- 11: Verriegelungsvorrichtung
- 12: Auslösetaster
- 13: Verbindungskabel
- 14: Hochspannungsstecker
- 15: Schreib-Lesevorrichtung
- 16: Handgriff

## Patentansprüche

1. Vorrichtung zur Erzeugung von Stoßwellen für die Behandlung am menschlichen oder tierischen Körper, umfassend eine Steuereinheit (1), einen Handapparat (2) und einen auswechselbaren Applikatorkopf (3) zur Erzeugung von Stoßwellen für die Behandlung am menschlichen oder tierischen Körper, wobei der Applikatorkopf (3) eine Reflexions- oder Fokussierungseinrichtung, zwei Elektroden (7a, 7b) zur Erzeugung einer Funkenstrecke sowie eine Membran (5), die mit der Reflexions- oder Fokussierungseinrichtung ein flüssiges Medium (6) einschließt, das geeignet ist beim Anlegen einer Spannung zwischen 1 kV und 30 kV ein Plasma zu erzeugen, umfasst,
wobei der Applikatorkopf (3) lösbar mit dem Handapparat (2) elektrisch und mechanisch verbunden werden kann
**dadurch gekennzeichnet, dass**
das Auswechseln des Applikatorkopfes (3) mittels einer im Handapparat (2) untergebrachten elektrisch-mechanischen, steuerbaren Verriegelungsvorrichtung (11) verhindert und freigegeben werden kann.

2. Vorrichtung nach Anspruch 1, **dadurch gekennzeichnet, dass** die Reflexions- oder Fokussierungseinrichtung (4) eine ellipsoide Geometrie aufweist.

3. Vorrichtung nach Anspruch 1, **dadurch gekennzeichnet, dass** die Reflexions- oder Fokussierungseinrichtung (4) eine paraboloide Geometrie aufweist.

4. Vorrichtung nach einem der vorhergehenden Ansprüche, **dadurch gekennzeichnet, dass** der Applikatorkopf (3) über ein Kommunikations-Speicher-Medium (8) verfügt, die es einer Steuereinheit (1) ermöglicht, den Applikatorkopf (3) zu identifizieren.

5. Vorrichtung nach Anspruch 4, **dadurch gekennzeichnet, dass** das Kommunikations-Speicher-Medium (8) über eine leitungsgebundene oder eine kontaktlose Verbindung mit der Steuereinheit (1) verbunden werden kann.

6. Vorrichtung nach Anspruch 4 oder 5, **dadurch gekennzeichnet, dass** der Datenaustauch zwischen Erkennungseinrichtung und Steuereinheit (1) über Radiofrequenzen erfolgt.

7. Vorrichtung nach einem der vorhergehenden Ansprüche, **dadurch gekennzeichnet, dass** die angelegte Spannung zur Erzeugung des Plasmas zwischen 5 kV und 30 kV, vorzugsweise zwischen 5 kV und 20 kV beträgt.

8. Vorrichtung nach einem der vorhergehenden Ansprüche, **dadurch gekennzeichnet, dass** der Applikatorkopf über eine lösbare Zündvorrichtung mit zwei Elektroden (7a, 7b) verfügt, die mit einer elektrisch-mechanischen Steckvorrichtung (9, 10) mit dem Handapparat (2) und einer Steuereinheit (1) verbunden ist.

9. Vorrichtung nach einem der vorhergehenden Ansprüche, **dadurch gekennzeichnet, dass** der Applikatorkopf (3) das flüssige Medium (6) vollständig einschließt, über keine permanente Verbindung zu einer externen Vorrichtung für das Spülen oder Wechseln des flüssigen Mediums (6) verfügt und nur als abgeschlossenen Einheit lösbar mit dem Handapparat (2) verbunden ist.

10. Vorrichtung nach einem der vorhergehenden Ansprüche, **dadurch gekennzeichnet, dass** der Handapparat über ein Verbindungskabel und einen Hochspannungsstecker mit der Steuereinheit verbunden ist.

11. Vorrichtung nach einem der vorhergehenden Ansprüche, **dadurch gekennzeichnet, dass** der Handapparat (2) über einen Taster (12) für die Auslösung von Stoßwellen verfügt, welcher mit der Steuereinheit (1) verbunden ist.

12. Vorrichtung nach einem der vorhergehenden Ansprüche, **dadurch gekennzeichnet, dass** der Applikatorkopf (3) über eine lösbare Verbindung mit der Steuereinheit (1) verbunden ist, die als tragbares Tischgerät ausgebildet ist.

## Claims

1. Apparatus for generating shockwaves for treatment on human or animal body, comprising a control unit (1), a handset (2) and a replaceable applicator head (3) for generating shockwaves for treatment on the human or animal body, wherein the applicator head (3) comprises a reflection or focusing device, two electrodes (7a, 7b) for generating a spark gap and a membrane (5) that with the reflection or focusing means encloses a liquid medium (6), which is suitable to produce a plasma when a voltage between 1 kV and 30 kV is applied, wherein the applicator head (3) can be detachably connected to the handset (2) electrically and mechanically, **characterised in that** the replacement of the applicator head (3) can be prevented and enabled electro-mechanically by means of a controllable locking device (11) accommodated in the handset (2).

2. Apparatus according to claim 1, **characterised in that** the reflection or focusing device (4) has an ellipsoidal geometry.

3. Device according to claim 1, **characterised in that** the reflection or focusing device (4) has a paraboloid geometry.

4. Device according to one of the preceding claims **characterised in that** the applicator head (3) has a communication memory medium (8) that allows a control unit (1) to identify the applicator head (3).

5. Device according to claim 4, **characterised in that** the communication memory medium (8) can be connected with the control unit (1) via a wired or a contactless connection.

6. Device according to claim 4 or 5, **characterised in that** the data exchange between detection device and control unit (1) occurs via radio frequencies.

7. Device according to one of the preceding claims **characterised in that** the applied voltage for generating the plasma is between 5 kV and 30 kV, preferably between 5 kV and 20 kV.

8. Device according to one of the preceding claims, **characterised in that** the applicator head has a releasable ignition device with two electrodes (7a, 7b) that is connected to the handset (2) and a control unit (1) by an electro-mechanical plug-in device (9, 10).

9. Device according to one of the preceding claims **characterised in that** the applicator head (3) completely encloses the liquid medium (6), has no permanent connection to an external device for rinsing or changing the liquid medium (6) and is only detachably connected to the handset (2) as a closed unit.

10. Device according to one of the preceding claims **characterised in that** the handset is connected to the control unit via a connecting cable and a high-voltage plug.

11. Device according to one of the preceding claims **characterised in that** the handset (2) has a button (12) for triggering shock waves, which is connected to the control unit (1).

12. Device according to one of the preceding claims **characterised in that** the applicator head (3) is attached to the control unit (1) via a releasable connection which is designed as a portable table device.

## Revendications

1. Dispositif servant à produire des ondes de choc pour le traitement du corps humain ou animal, comprenant une unité de commande (1), un appareil à main (2) et une tête d'applicateur (3) pouvant être remplacée servant à produire des ondes de choc pour le traitement du corps humain ou animal, dans lequel la tête d'applicateur (3) comprend un système réfléchissant ou de concentration, deux électrodes (7a, 7b) servant à produire un éclateur à étincelles ainsi qu'une membrane (5), qui renferme un milieu liquide (6) avec le système réfléchissant ou de concentration, qui est adapté pour produire un plasma lors de l'application d'une tension entre 1 kV et 30 kV,
dans lequel la tête d'applicateur (3) peut être reliée électriquement ou mécaniquement de manière amovible à l'appareil à main (2),
**caractérisé en ce que**
le remplacement de la tête d'applicateur (3) peut être empêché et débloqué au moyen d'un dispositif de verrouillage (11), abrité dans l'appareil à main (2), électromécanique et pouvant être commandé.

2. Dispositif selon la revendication 1, **caractérisé en ce que** le système réfléchissant ou de concentration (4) présente une géométrie ellipsoïdale.

3. Dispositif selon la revendication 1, **caractérisé en ce que** le système réfléchissant ou de concentration (4) présente une géométrie paraboloïde.

4. Dispositif selon l'une quelconque des revendications précédentes, **caractérisé en ce que** la tête d'applicateur (3) dispose d'un support de stockage de communication (8), qui permet à une unité de commande (1) d'identifier la tête d'applicateur (3).

5. Dispositif selon la revendication 4, **caractérisé en ce que** le support de stockage de communication (8) peut être relié à l'unité de commande (1) par l'intermédiaire d'une liaison filaire ou sans contact.

6. Dispositif selon la revendication 4 ou 5, **caractérisé en ce que** l'échange de données entre le système d'identification et l'unité de commande (1) s'effectue par l'intermédiaire de radiofréquences.

7. Dispositif selon l'une quelconque des revendications précédentes, **caractérisé en ce que** la tension appliquée servant à produire le plasma est entre 5 kV et 30 kV, de préférence entre 5 kV et 20 kV.

8. Dispositif selon l'une quelconque des revendications précédentes, **caractérisé en ce que** la tête d'applicateur dispose d'un dispositif d'allumage amovible avec deux électrodes (7a, 7b), qui est relié à un dispositif d'enfichage (9, 10) électromécanique avec l'appareil à main (2) et une unité de commande (1).

9. Dispositif selon l'une quelconque des revendications précédentes, **caractérisé en ce que** la tête d'applicateur (3) renferme en totalité le milieu liquide (6), ne dispose d'aucune liaison permanente avec un dispositif externe pour le rinçage ou l'échange du milieu liquide (6) et est reliée seulement en tant qu'unité hermétiquement fermée de manière amovible à l'appareil à main (2).

10. Dispositif selon l'une quelconque des revendications précédentes, **caractérisé en ce que** l'appareil à main est relié à l'unité de commande par l'intermédiaire d'un câble de liaison et d'un connecteur enfichable haute tension.

11. Dispositif selon l'une quelconque des revendications précédentes, **caractérisé en ce que** l'appareil à main (2) dispose d'un bouton-poussoir (12) pour le déclenchement d'ondes de choc, qui est relié à l'unité de commande (1).

12. Dispositif selon l'une quelconque des revendications précédentes, **caractérisé en ce que** la tête d'applicateur (3) est reliée par l'intermédiaire d'une liaison amovible à l'unité de commande (1), qui est réalisée en tant qu'appareil de table portatif.
